Europäisches Patentamt

European Patent Office

Office européen des brevets

(11) Veröffentlichungsnummer: **0 404 997**

**A1**

## (12) EUROPÄISCHE PATENTANMELDUNG

(21) Anmeldenummer: 89111990.1

(22) Anmeldetag: 30.06.89

(51) Int. Cl.⁵: **A61B 6/04**

(43) Veröffentlichungstag der Anmeldung:
**02.01.91 Patentblatt 91/01**

(84) Benannte Vertragsstaaten:
**DE FR GB NL**

(71) Anmelder: **Siemens Aktiengesellschaft**
**Wittelsbacherplatz 2**
**D-8000 München 2(DE)**

(72) Erfinder: **Schäfer, Willi, Dipl.-Ing.(FH)**
**Genglerstrasse 20**
**D-8520 Erlangen(DE)**

(54) **Patientenlagerungseinrichtung mit einer schwenkbaren Patientenlagerungsplatte.**

(57) Die Erfindung betrifft eine Patientenlagerungseinrichtung mit einer um eine horizontale Schwenkachse (S) schwenkbar an einem Stützteil (3) angebrachten Patientenlagerungsplatte (1). Dabei ist die Patientenlagerungsplatte (1) mit dem Stützteil (3) mittels zweier geradliniger Längsführungen (4) verbunden. Die Mittelachsen (A) der Längsführungen (4) sind jeweils in einer rechtwinklig zur Schwenkachse (S) verlaufenden Ebene (E) angeordnet, schließen miteinander einen Winkel ("Alpha") ein, der kleiner als 180° ist, und sind jeweils um eine parallel zur Schwenkachse (S) verlaufende Achse (B) schwenkbar.

FIG 1

## PATIENTENLAGERUNGSEINRICHTUNG MIT EINER SCHWENKBAREN PATIENTENLAGERUNGSPLATTE

Die Erfindung betrifft eine Patientenlagerungseinrichtung mit einer um eine vorzugsweise wenigstens im wesentlichen horizontal verlaufende Schwenkachse schwenkbar an einem Stützteil angebrachten Patientenlagerungplatte.

Derartige Patientenlagerungseinrichtungen werden beispielsweise in der Röntgendiagnostik, der Strahlentherapie, der Urologie und der Chirurgie benötigt, um einen Patienten für diagnostische oder therapeutische Zwecke in geeigneter Weise positionieren zu können.

Eine Patientenlagerungseinrichtung der eingangs genannten Art ist in der Druckschrift der Fa. Siemens "SIREGRAPH D1", Best.-Nr. A91001-M1018-G531-01, im Zusammenhang mit einem Röntgendiagnostikgerät beschrieben. Dabei ist die Patientenlagerungsplatte an ihrer Unterseite mit einem kreissegmentförmigen Bauteil versehen, das in einer entsprechenden Lagerungseinrichtung eines als Stützteil für die Patientenlagerungsplatte dienenden Gerätefußes derart abrollend aufgenommen ist, daß die Patientenlagerungsplatte um eine durch den Mittelpunkt des kreissegmentförmigen Bauteiles verlaufende horizontale Schwenkachse schwenkbar ist. Dadurch ist die Patientenlagerungsplatte selbst bei Schwenkwinkeln von mehr als 180° - derart große Schwenkwinkel sind aber nur in bestimmten Fällen erforderlich -mit hoher Präzision zu schwenken. Allerdings ist mit der bekannten Lösung neben einem hohen Bauraumbedarf auch ein ganz erheblicher konstruktiver und fertigungstechnischer Aufwand verbunden, der entsprechende Kosten nach sich zieht.

Der Erfindung liegt die Aufgabe zugrunde, eine Patientenlagerungseinrichtung der eingangs genannten Art so auszubilden, daß bei geringem Bauraumbedarf und geringem konstruktiven, fertigungstechnischen und finanziellen Aufwand eine ausreichend präzise Schwenkbarkeit der Patientenlagerungsplatte bei Schwenkwinkeln in der Größenordnung von etwa ± 20° gewährleistet ist.

Nach der Erfindung wird diese Aufgabe durch die folgenden Merkmale gelöst:

a) die Patientenlagerungsplatte ist mit dem Stützteil mittels zweier Längsführungen verbunden, wobei die Längsführungen jeweils ein geradliniges Führungsteil und ein mit dem Führungsteil derart zusammenwirkendes Tragteil aufweisen, daß das Führungsteil und das Tragteil längs der geradlinigen Mittelachse der Längsführung relativ zueinander verschiebbar sind,

b) die Mittelachsen der Längsführungen sind jeweils in einer rechtwinklig zur Schwenkachse verlaufenden Ebene angeordnet,

c) die Mittelachsen der Längsführungen schließen miteinander einen Winkel ein, der kleiner als 180° ist, und

d) die Mittelachsen der Längsführungen sind jeweils um eine parallel zur Schwenkachse verlaufende Achse schwenkbar.

Infolge der Verwendung von Längsführungen, es kommen hier z.B. handelsübliche Bauteile in Frage, erfordert die erfindungsgemäße Lösung nur einen geringen konstruktiven, fertigungstechnischen und finanziellen Aufwand. Außerdem ist der Bauraumbedarf im Vergleich zum Stand der Technik erheblich geringer. Allerdings ist im Falle der Erfindung keine definierte, während des Schwenkvorganges stationäre Schwenkachse vorhanden. Vielmehr soll diejenige rechtwinklig zu den Ebenen der Mittelachsen der Längsführungen verlaufende Achse als Schwenkachse im Sinne der Erfindung verstanden werden, für die während des Schwenkvorganges die geringsten Verlagerungen auftreten. Bei einem gemäß der Erfindung ausgeführten Prototypen mit horizontaler Schwenkachse ergab sich bei Schwenkwinkeln von ± 15° lediglich eine Verlagerung der Schwenkachse in der Größenordnung von ± 15 mm in horizontaler Richtung und + 11 mm in vertikaler Richtung, was in der Regel tolerierbar ist.

Für Fälle, in denen ein innerhalb eines auf der Patientenlagerungsplatte liegenden Patienten befindlicher therapeutisch und/ oder diagnostisch relevanter Bereich während des Schwenkens der Patientenlagerungsplatte seine Lage beibehalten soll, ist gemäß einer bevorzugten Variante der Erfindung vorgesehen, daß die Schwenkachse oberhalb der Patientenlagerungsplatte und innerhalb des durch die Mittelachsen der Längsführungen eingeschlossenen Winkels verläuft. Der Patient muß dann so auf der Patientenlagerungsplatte gelagert werden, daß die Schwenkachse durch den diagnostisch oder therapeutisch relevanten Bereich verläuft.

Obwohl infolge des Umstandes, daß die Mittelachsen der Längsführungen einen von 180° abweichenden Winkel einschließen, an sich bereits dann, wenn sich die Mittelachsen der Längsführungen in einer gemeinsamen Ebene befinden, eine stabile Unterstützung der Patientenlagerungsplatte gewährleistet ist, ist im Interesse einer erhöhten Stabilität gemäß einer Ausführungsform der Erfindung vorgesehen, daß die die Mittelachsen der Längsführungen enthaltenden Ebenen im Abstand voneinander angeordnet sind. Ebenfalls im Interesse einer erhöhten Stabilität sind im Falle einer weiteren Ausführungsform der Erfindung mehr als zwei Längsführungen vorgesehen, wobei die Mittelachsen der Längsführungen in zwei parallel zur Schwenkachse verlaufenden, miteinander den Winkel einschließenden Ebenen angeordnet sind.

Eine bevorzugte Ausführungsform der Erfindung sieht vor, daß jeweils zwei Längsführungen, deren Mittelachsen miteinander den Winkel einschließen und in einer gemeinsamen Ebene angeordnet sind, ein Paar von Längsführungen bilden. Dabei ist es im Interesse einer hohen Stabilität zweckmäßig, wenn wenigstens zwei Paare von Längsführungen vorgesehen sind, deren Mittelachsen jeweils den gleichen Winkel einschließen.

Eine Ausführungsform der Erfindung sieht vor, daß die Achsen, um die die Mittelachsen der Längsführungen schwenkbar sind, jeweils die Mittelachse der entsprechenden Längsführung schneiden. Hierdurch wird der Vorteil erzielt, daß die Patientenlagerungsplatte und das Stützteil beim Schwenken in jeder Position parallel zueinander stehen. Der Störkantenbereich zwischen Patientenlagerungsplatte und Stützteil bleibt weitgehend konstant. Außerdem werden zusätzliche Drehmomente um die Mittelachse der Längsführungen vermieden. Die erforderliche Schwenkbarkeit der Mittelachsen der Längsführungen ist mit konstruktiv geringem Aufwand realisierbar, wenn gemäß einer Ausführungsform der Erfindung die Tragteile der Längsführungen um die Achsen schwenkbar sind.

Gemäß einer bevorzugten Ausführungsform der Erfindung ist vorgesehen, daß die Führungsteile mit der Patientenlagerungsplatte und die Tragteile mit dem Stützteil verbunden sind. Im Vergleich zum umgekehrten Fall ergibt sich der Vorteil, daß die geometrischen Gesamtabmessungen, also das umbaute Volumen, der Patientenlagerungseinrichtung mit der schwenkbaren Patientenlagerungsplatte wesentlich kleiner sind.

Eine weitere Variante der Erfindung sieht vor, daß die Führungsteile durch Führungsschienen und die Tragteile durch die Führungsschienen aufnehmende Lagerungseinrichtungen gebildet sind. Es können dann beispielsweise von der Fa. Star Kugelhalter, Schweinfurt, DE, unter der Bezeichnung "STAR" 1601-15 vertriebene Längsführungen verwendet werden.

Ausführungsbeispiele der Erfindung sind in den beigefügten Zeichnungen dargestellt. Es zeigen:

Fig. 1 eine Stirnansicht einer erfindungsgemäßen Patientenlagerungseinrichtung,

Fig. 2 eine Seienansicht der Patientenlagerungseinrichtung nach Fig. 1,

Fig. 3 eine Stirnansicht einer weiteren erfindungsgemäßen Patientenlagerungseinrichtung, und

Fig. 4 in vergrößerter Darstellung einen Schnitt gemäß der Linie IV-IV in Fig. 3.

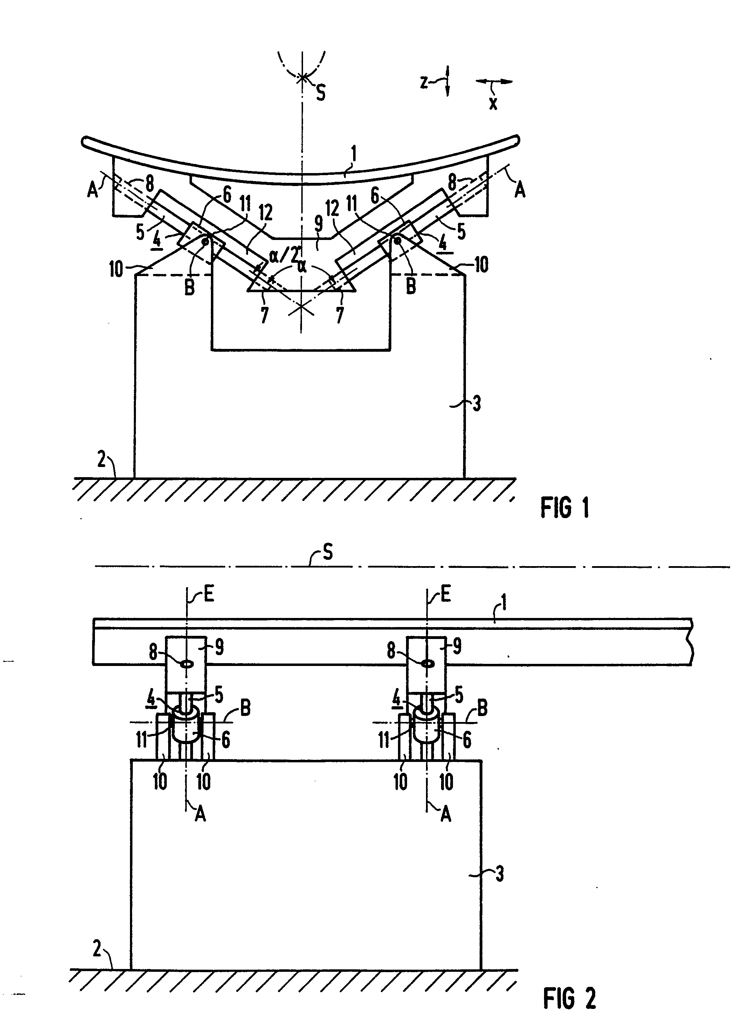

In den Fig. 1 und 2 ist die Erfindung anhand eines Patientenlagerungstisches dargestellt, der eine Patientenlagerungsplatte 1 aufweist, die um eine oberhalb der Patientenlagerungsplatte 1 parallel zur Längsachse des Patientenlagerungstisches verlaufende Schwenkachse S schwenkbar mit einem als Stützteil für die Patientenlagerungsplatte 1 wirkenden, auf dem Boden 2 ruhenden Sockel 3 verbunden ist.

Die Verbindung der Patientenlagerungsplatte 1 mit dem Sockel 3 erfolgt mit Hilfe von vier Längsführungen 4, die jeweils ein geradliniges Führungsteil 5 in Form einer zylindrischen Stange und ein das Führungsteil 5 aufnehmendes Tragteil 6 mit einer dem Querschnitt des Führungsteiles 5 angepaßten Bohrung aufweisen. Bei den Tragteilen 6 kann es sich beispielsweise um hülsenförmige Gleitlager oder um hülsenförmige Längskugellager, sogenannte Kugelhülsen, handeln, wie sie beispielsweise in der DE-OS 19 49 182 beschrieben sind. Dabei wirken die Führungsteile 5 mit den jeweiligen Tragteilen 6 derart zusammen, daß die Führungsteile 5 und die Tragteile 6 jeweils längs der geradlinigen Mittelachsen A der Längsführungen 4 relativ zueinander verschiebbar sind.

Die Führungsteile 5 der Längsführungen 4 sind mit der Patientenlagerungsplatte 1 dadurch verbunden, daß die Führungsteile 5 jeweils zweier Längsführungen 4 in den miteinander fluchtenden Bohrungen 7, 8 zweier quer zur Längsachse der Patientenlagerungsplatte 1 verlaufend an der Patientenlagerungsplatte 1 angebrachter Traversen 9 axial unverschieblich aufgenommen sind. Die Tragteile 6 der Längsführungen 4 sind mit dem Sockel 3 verbunden, indem sie in an dem Sockel 3 angebrachten Gabelteilen 10 mit Hilfe von Bolzen 11 schwenkbar gelagert sind. Dabei ver laufen die Achsen B der Bolzen 11 parallel zur Schwenkachse S und schneiden außerdem die Mittelachse A der jeweiligen Längsführung 4. Die Traversen 9 sind mit Ausschnitten 12 versehen, die eine freie Verschiebbarkeit der Tragteile 6 gewährleisten.

Die Bohrungen 7, 8 der Traversen 9 verlaufen derart, daß die Mittelachsen A der beiden Längsführungen 4, deren Führungsteile 5 jeweils in einer Traverse 9 aufgenommen sind, einen Winkel "Alpha" miteinander einschließen, der kleiner als 180° ist, wobei die Schwenkachse S innerhalb des durch die Mittelachsen A der Längsführungen 4 eingeschlossenen Winkels Alpha verläuft. Außerdem sind die Traversen 9 jeweils derart an der Patientenlagerungsplatte 1 angebracht und verlaufen die Bohrungen 7, 8 der Traversen derart, daß die Mittelachsen A beider zu einer Traverse 9 gehöriger Längsführungen 4, die den Winkel "Alpha" miteinander einschließen, in einer gemeinsamen, rechtwinklig zur Schwenkachse S verlaufenden Ebene E angeordnet sind. Die zwei zu einer Traverse 9 gehörigen Längsführungen 4 bilden also ein Paar von Längsführungen 4. Dabei sind die Winkel "Alpha", die die Mittelachsen A der Längsführungen 4 der beiden Paare von Längsführungen 4 jeweils miteinander einschließen, gleich groß. Die in Fig. 1 gezeigte Stirnansicht gilt übrigens unabhängig von der gewählten Blickrichtung. Die Mittelachsen A der Längsführungen 4 liegen dann also in zwei parallel zur Schwenkachse S verlaufenden, miteinander den Winkel "Alpha" einschließenden Ebenen, wobei jede Ebene die Mittelachsen A zweier zu unterschiedlichen Paaren gehöriger Längsführung 4 enthält. Die die Mittelachsen A der Längsführungen 4 enthaltenden Ebenen E sind im Abstand voneinander angeordnet.

Infolge der beschriebenen Ausbildung der erfindungsgemäßen Patientenlagerungseinrichtung ist die Patientenlagerungsplatte 1 wie erwähnt um eine parallel zur Längsachse der Patientenlagerungsplatte 1 und oberhalb derselben verlaufende Schwenkachse S schwenkbar. Dabei handelt es sich jedoch nicht um eine definierte, während des Schwenkvorganges stationäre Schwenkachse. Vielmehr soll unter der Schwenkachse S hier diejenige Achse verstanden werden, die senkrecht zu den Ebenen E verläuft und die Winkelhalbierende der Winkel "Alpha" schneidet und während des Schwenkvorganges die geringste Verlagerung erfährt. Beim Schwenken der Patientenlagerungsplatte 1 verschieben sich die Führungsteile 5 in den Tragteilen 6, die dabei eine Schwenkbewegung um die Achsen B ausführen. Dabei verlagert sich die Schwenkachse S, die bei ungeschwenkter Patientenlagerungsplatte 1 die in Fig. 1 dargestellte Position einnimmt, entlang der in Fig. 1 strichpunktiert eingetragenen Bahn. Dabei ist der Verlauf der Bahn lediglich qualitativ und nicht maßstäblich zu verstehen. Bei einem entsprechend den Fig. 1 und 2 ausgeführten Prototypen, bei dem der Winkel "Alpha" 128° und der Abstand der Achsen B der Paare von Längsführungen 4 jeweils 310 mm beträgt, liegt die Schwenkachse 374 mm oberhalb der Schnittpunkte der Mittelachsen A der Längsführungen 4. Für Schwenkwinkel von ± 15° tritt bei dem Prototyp eine Verlagerung der Schwenkachse S in Richtung des Pfeiles z von nicht mehr als + 11 mm und in Richtung des Pfeiles y von nicht mehr als ± 15 mm auf. Die Verlagerung der Schwenkachse S ist in der Praxis also in der Regel vernachlässigbar.

Infolge des Umstandes, daß die die Mittelachsen der Längsführungen 4 enthaltenden Ebenen E bei dem Prototypen in einem Abstand von mehreren Hundert Millimetern voneinander angeordnet sind, und infolge der Verwendung von zwei Paaren von Längsführungen 4 wird eine mechanisch stabile Unterstützung der Patientenlagerungsplatte 1 erreicht.

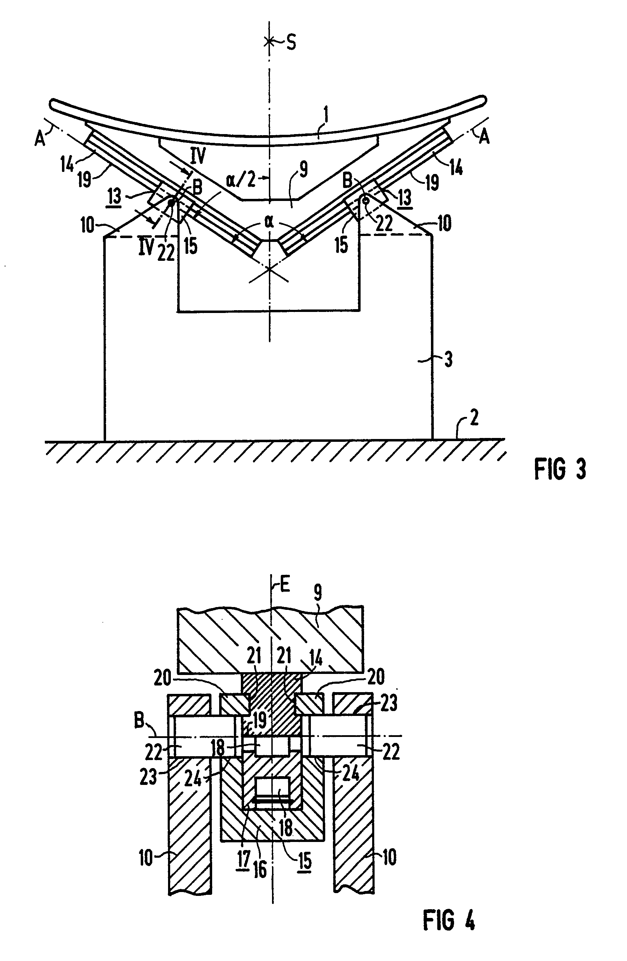

Das Ausführungsbeispiel gemäß den Fig. 3 und 4 unterscheidet sich von dem zuvor beschriebenen im wesentlichen nur durch die andersartige Ausbildung der hier vorgesehenen Längsführungen 13, weshalb für gleiche Teile jeweils die gleichen Bezugszeichen wie zuvor verwendet werden. Die Längsführungen 13 weisen als Führungsteil jeweils eine gerade Führungsschiene 14 von etwa doppel-T-förmigem Querschnitt auf. Im Falle des beschriebenen Ausführungsbeispieles sind die Führungsschienen 14 in nicht dargestellter Weise mit den Traversen 9 verschraubt. Es kommen jedoch auch andere geeignete Befestigungsarten in Frage. Außerdem können die Führungsschienen 14 einstückig mit den Traversen 9 ausgeführt sein. Als Tragteil weisen die Längsführungen 13 jeweils einen Lagerungsschuh 15 auf. Dieser besitzt einen U-förmigen Grundkörper 16, zwischen dessen Schenkeln ein Längswälzlager 17 mit zylindrischen Wälzkörpern 18 befestigt ist. Derartige auch als Rollenumlaufschuhe bezeichnete Längswälzlager können z.B. von der Firma SKF, Schweinfurt, DE, bezogen werden. Die Wälzkörper 18 der Längswälzlager 17 rollen auf der als ebene Lauffläche 19 ausgebildeten Außenseite des von der Traverse 9 gewandten Flansches der Führungsschiene 14 ab. Dabei stellt jeweils die Mittellinie der Lauffläche 19 die Mittelachse A einer Längsführung 13 dar. Die Schenkel des U-förmigen Grundkörpers 16 sind an ihren freien Enden mit nach innen gerichteten Vorsprüngen 20 versehen, die in durch die Innenflächen der Flansche der Führungsschiene 14 und die Seitenflächen des Steges der Führungsschiene 14 begrenzten Nuten 21 eingreifen. Beim Schwenken der Lagerungsplatte 1 um die Schwenkachse S rollen also die Wälzkörper 18 der Längswälzlager 17 auf den Laufflächen 19 der Führungsschienen 14 ab. Gleichzeitig gleiten die Vorsprünge 20 in den Nuten 21. Die Lagerungsschuhe 15 umgreifen also die Führungsschienen 14 formschlüssig derart, daß Kräfte in beliebigen Richtungen übertragen werden können.

Die Lagerungsschuhe 15 sind in den Gabelteilen 10 mit Hilfe von Bolzen 22, die in entsprechende Bohrungen 23, 24 der Gabelteile 10 bzw. der Grundkörper 16 der Lagerungsschuhe 15 aufgenommen sind, gelenkig gelagert, und zwar derart, daß die Laufflächen 19 der Führungsschienen 14 die Achsen B enthalten, die den Mittelachsen der Bolzen 22 entsprechen.

Die Ausführungsbeispiele betreffen ausschließlich solche Patientenlagerungseinrichtungen, bei denen die Patientenlagerungsplatte 1 um eine parallel zu ihrer Längsachse verlaufende Schwenkachse S schwenkbar ist. Die Erfindung kann jedoch auch bei Patientenlagerungseinrichtungen Verwendung finden, deren Patientenlagerungsplatte um eine andere, z.B. eine quer zur Längsachse der Lagerungsplatte verlaufende Schwenkachse schwenkbar ist. Außerdem kann anders als im Falle der beschriebenen Ausführungsbeispiele vorgesehen sein, daß die Führungsteile 5 bzw. 14 der Längsführungen 4 bzw. 13 an dem Sockel 3 und die Tragteile 6 bzw. 15 an der Patientenlagerungsplatte 1 oder den Traversen 9 angebracht sind.

**Ansprüche**

1. Patientenlagerungseinrichtung mit einer um eine vorzugsweise wenigstens im wesentlichen horizontal verlaufende Schwenkachse (S) schwenkbar an einem Stützteil (3) angebrachten Patientenlagerungsplatte (1), aufweisend folgende Merkmale:

    a) die Patientenlagerungsplatte (1) ist mit dem Stützteil (3) mittels zweier Längsführungen (4, 13) verbunden, wobei die Längsführungen (4, 13) jeweils ein geradliniges Führungsteil (5, 14) und ein mit dem Führungsteil (5, 14) derart zusammenwirkendes Tragteil (6, 15) aufweisen, daß das Führungsteil (5, 14) und das Tragteil (6, 15) längs der geradlinigen Mittelachse (A) der Längsführung (4, 13) relativ zueinander verschiebbar sind,

    b) die Mittelachsen (A) der Längsführungen (4, 13) sind jeweils in einer rechtwinklig zur Schwenkachse (S) verlaufenden Ebene (E) angeordnet,

    c) die Mittelachsen (A) der Längsführungen (4, 13) schließen miteinander einen Winkel "Alpha" ein, der kleiner als 180° ist, und

    d) die Mittelachsen (A) der Längsführungen (4, 13) sind jeweils um eine parallel zur Schwenkachse (S) verlaufende Achse (B) schwenkbar.

2. Patientenlagerungseinrichtung nach Anspruch 1, **dadurch gekennzeichnet,** daß die Schwenkachse (S) oberhalb der Patientenlagerungsplatte (1) und innerhalb des durch die Mittelachsen (A) der Längsführungen (4, 13) eingeschlossenen Winkels ("Alpha") verläuft.

3. Patientenlagerungseinrichtung nach Anspruch 1 oder 2, **dadurch gekennzeichnet,** daß die die Mittelachsen (A) der Längsführungen (4, 13) enthaltenden Ebenen (E) im Abstand voneinander angeordnet sind.

4. Patientenlagerungseinrichtung nach einem der Ansprüche 1 bis 3, **dadurch gekennzeichnet,** daß mehr als zwei Längsführungen (4, 13) vorgesehen sind, wobei die Mittelachsen (A) der Längsführungen (4, 13) in zwei parallel zur Schwenkachse (S) verlaufenden, miteinander den Winkel ("Alpha") einschließenden Ebenen angeordnet sind.

5. Patientenlagerungseinrichtung nach einem der Ansprüche 1 bis 4, **dadurch gekennzeichnet,** daß jeweils zwei Längsführungen (4, 13), deren Mittelachsen (A) miteinander den Winkel ("Alpha") einschließen und in einer gemeinsamen Ebene (E) angeordnet sind, ein Paar von Längsführungen (4, 13) bilden.

6. Patientenlagerungseinrichtung nach Anspruch 5, **dadurch gekennzeichnet,** daß wenigstens zwei Paare von Längsführungen (4, 13) vorgesehen sind, deren Mittelachsen (A) jeweils den gleichen Winkel ("Alpha") einschließen.

7. Patientenlagerungseinrichtung nach einem der Ansprüche 1 bis 6, **dadurch gekennzeichnet,** daß die Achsen (B), um die die Mittelachsen (A) der Längsführungen (4, 13) schwenkbar sind, jeweils die Mittelachse (A) der entsprechenden Längsführung (4, 13) schneiden.

8. Patientenlagerungseinrichtung nach einem der Ansprüche 1 bis 7, **dadurch gekennzeichnet,** daß die Tragteile (6, 15) der Längsführungen (4, 13) um die Achsen (B) schwenkbar sind.

9. Patientenlagerungseinrichtung nach einem der Ansprüche 1 bis 8, **dadurch gekennzeichnet,** daß die Führungsteile (5, 14) mit der Patientenlagerungsplatte (1) und die Tragteile (6, 15) mit dem Stützteil (3) verbunden sind.

10. Patientenlagerungseinrichtung nach einem der Ansprüche 1 bis 9, **dadurch gekennzeichnet,** daß die Führungsteile durch Führungsschienen (14) und die Tragteile durch die Führungsschienen (14) formschlüssig umgreifende Lagerungsschuhe (15) gebildet sind.

FIG 1

FIG 2

FIG 3

FIG 4

| Europäisches Patentamt | EUROPÄISCHER RECHERCHENBERICHT | Nummer der Anmeldung |
| --- | --- | --- |
| | | EP 89 11 1990 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| --- | --- | --- | --- |
| A | US-A-3149229 (F.A.M. MOREL)<br>* Spalte 1, Zeilen 63 - 66 *<br>* Spalte 3, Zeile 5 - Spalte 4, Zeile 49;<br>Figuren 1-5 *<br>--- | 1 | A61B6/04 |
| A | DE-A-2636746 (MALESKI-MAYSKA)<br>* das ganze Dokument *<br>--- | 1 | |
| A | FR-A-1191471 (SIEMENS AG)<br>* das ganze Dokument *<br>--- | 1 | |
| A | US-A-3609357 (WILLIAM E.M. JONES)<br>* das ganze Dokument *<br>----- | 1 | |
| | | | **RECHERCHIERTE SACHGEBIETE (Int. Cl.5)**<br><br>A61B<br>A61G<br>A47B |

Der vorliegende Recherchenbericht wurde für alle Patentansprüche erstellt

| Recherchenort | Abschlußdatum der Recherche | Prüfer |
| --- | --- | --- |
| DEN HAAG | 02 FEBRUAR 1990 | FERRIGNO A. |